# EUROPEAN PATENT SPECIFICATION

(11) **EP 1 555 994 B1**
(45) Date of publication and mention of the grant of the patent: **06.08.2014**
(21) Application number: 03754511.8
(22) Date of filing: 11.09.2003
(51) Int. Cl.: A61Q 11/00, A61K 8/02, A61K 8/19, A61K 8/22

(54) **TOOTH WHITENING STRIPS**
STREIFEN ZUM BLEICHEN DER ZÄHNE
BANDES DE BLANCHIMENT DES DENTS

(30) Priority: 11.09.2002 US 409862 P
(43) Date of publication of application: 27.07.2005
(60) Divisional application: 12008640.0
(73) Proprietor: THE PROCTER & GAMBLE COMPANY, Cincinnati, OH 45202 (US)
(72) Inventor: SAGEL, Paul, Albert, Maineville, OH 45039 (US); BARRON, Robert, Eugene, Cincinnati, OH 45231 (US); GERLACH, Robert, Woodrow, Wyoming, OH 45215 (US); SCOTT, Douglas, Craig, Loveland, OH 45140 (US); MCCLANAHAN, Stephen, Francis, Loveland, OH 45140 (US)
(74) Representative: Töpert, Verena Clarita
(86) International application number: PCT/US2003/028612
(87) International publication number: WO 2004/024104

(56) References cited:
- WO-A-01/68045
- US-A1- 2002 012 685
- US-B1- 6 419 906

## Description

### Technical Field of Invention

The present invention relates to strips for whitening teeth, and, more particularly, to strips for whitening teeth that have a whitening agent incorporated therein.

### Background of the Invention

Tooth whitening has become very popular over the past few years. More and more consumers are choosing to whiten their teeth. Options for tooth whitening include toothpastes, mouthrinses, chewing gums, in-office bleaching, and most commonly tooth whitening solutions used with a tray obtained either over-the-counter or from a dentist. Tooth whitening products using a strip of material in combination with a chemical whitening active are known in the art. For example, US patent no. 6,419,906 describes a tooth whitening product comprising a strip of material formed from a water hydratable polymer and a tooth whitening agent. While these whitening products may function for their intended purpose, there is continuing desire to improve the efficacy, stability, and aesthetics of these tooth whitening products.

US 6 419 906 B1 discloses a thin, flexible film which when applied to stained teeth is hydrated by saliva and is effective in such form to whiten teeth. The film comprises an anhydrous water hydratable ethylene oxide polymer matrix containing a solid peroxide whitening agent. Upon application to stained tooth surfaces, the peroxide whitening agent is solublilized by saliva present in the oral cavity into active whitening activity when the film is positioned and placed on the teeth.

US 2002/012685 Al teaches methods and delivery systems for whitening teeth. The methods include the steps of providing a flexible strip of material, applying a layer of a tooth whitening substance having a whitening active to a front surface of a plurality of teeth, conforming the strip of material to the front surface of the plurality of teeth, and conforming the strip of material to interstitial spaces between the plurality of teeth. The strip of material serves as a protective barrier for the tooth whitening substance for a sufficient period of time for the tooth whitening active to act upon the plurality of teeth. The delivery systems include a strip of material sized to cover a front surface of a plurality of teeth, wherein the strip of material is substantially flat and has sufficient flexibility to conform to the front surfaces of the plurality of teeth and to interstitial spaces there between during use. The delivery systems further include a layer of a tooth whitening substance in contact with the strip of material.

### Summary of the Invention

A tooth whitening product is provided which includes a web formed from a plurality of fibers which are arranged to provide a plurality of void spaces. A water hydratable polymer coating having a tooth whitening agent is deposited on the web.

### Brief Description of the Drawings

While the specification concludes with claims particularly pointing out and distinctly claiming the invention, it is believed that the present invention will be better understood from the following description taken in conjunction with the accompanying drawings in which:
Fig. 1 is a cross-sectional side view of tooth whitening product, not in accordance with the present invention, wherein a coating of a tooth whitening agent has been applied to a film;
Fig. 2 is a perspective view of an embodiment of a tooth whitening product of the present invention, wherein the tooth whitening product comprises a web and a coating that completely bridges the void spaces of the web and wherein a portion of the coating has been removed to reveal features there below;
Fig. 3 is a cross-sectional side view of the tooth whitening product of Fig. 2, wherein the void spaces are shown as partially filled;
Fig. 4 is a cross-sectional side view of the tooth whitening product of Fig. 2, wherein the void spaces are shown as completely filled;
Fig. 5 is a perspective view of another embodiment of a tooth whitening product of the present invention, wherein the tooth whitening product comprises a web and a coating that partially bridges the void spaces of the web;
Fig. 6 is a perspective view of another embodiment of a tooth whitening product of the present invention, wherein the tooth whitening product comprises a web and a coating that does not bridge the void spaces of the web;

### Detailed Description of the Embodiments

Reference will now be made in detail to the present preferred embodiments of the invention, examples of which are illustrated in the accompanying drawings wherein like numerals indicate the same elements throughout the views and wherein elements having the same two last digits (e.g., 20 and 120) connote similar elements. The present invention is directed to tooth whitening products in the form of a film or strip and comprising a tooth whitening agent and a water hydratable polymer. Preferably, the water hydratable polymer is also at least partially water soluble (i.e., only a portion of an added polymer dissolves into water). While the present invention will be described herein with respect to these films or strips, it is contemplated that the present invention can be used with other tooth whitening products, such as dental trays. Water hydratable polymers suitable for use with the present invention include ethylene oxide polymers, homopolymers or mixtures of ethylene oxide polymers of varying molecular weight ranging from about 10,000 Daltons and up to about 10,000,000 Daltons and preferably in the range of about 100,000 to about 1,500,000 Daltons. Such ethylene oxide polymers are commercially available from various sources. Polyethylene oxide in the molecular weight range of 10,000 to 1,000,000 Daltons is available from the Union Carbide Company under the tradename "Polyox". Other water hydratable polymers include polypropylene oxide, polyethylene oxide, Carbopol, polyvinyl alcohol, ethyl vinyl acetate, sodium alginate, methyl methacrylate, xanthan gum, pectin, pullulan, guar gum, agar, polyvinyl pyrolidone (PVP), carrageanan, celluloses (e.g., hydroxypropylcellulose, hydroxyethyl cellulose, hydroxypropylmethyl cellulose and hydroxymethyl cellulose), and mixtures thereof. While the thickness of the film may vary, as described more fully hereafter, the film may have a thickness between 0.1 micrometer and 1500 micrometer (*µ*m).

Hydration of the water hydratable polymer by saliva in the oral cavity solubilizes the whitening agent incorporated in the polymer matrix. The whitening agent is then released from the tooth whitening product to the tooth surfaces to which the film is applied. Whitening agents suitable for the practice of the present invention include peroxides, metal chlorites, perborates, percarbonates, peroxyacids, persulfates, and combinations thereof. Suitable peroxide compounds include hydrogen peroxide, urea peroxide, calcium peroxide, and mixtures thereof. Suitable metal chlorites include calcium chlorite, barium chlorite, magnesium chlorite, lithium chlorite, sodium chlorite, and potassium chlorite. The preferred chlorite is sodium chlorite. A preferred percarbonate is sodium percarbonate. Preferred persulfates are oxones.

Optionally, the water hydratable polymer may be mixed with a plasticizer. Suitable plasticizers include glycols such as propylene glycol, polyethylene glycol, polyhydric alcohols such as glycerin and sorbitol and glycerol esters such as glycerol triacetate. Glycerin can be used as well as propylene glycol or polyethylene glycol such as is available from Union Carbide Corporation as their series of Carbowaxes that range in molecular weight from 200 to 600 Daltons. Other plasticizers include cellulose esters, sebacate esters, castor oil, tricresyl phosphate, and pthalate adipate.

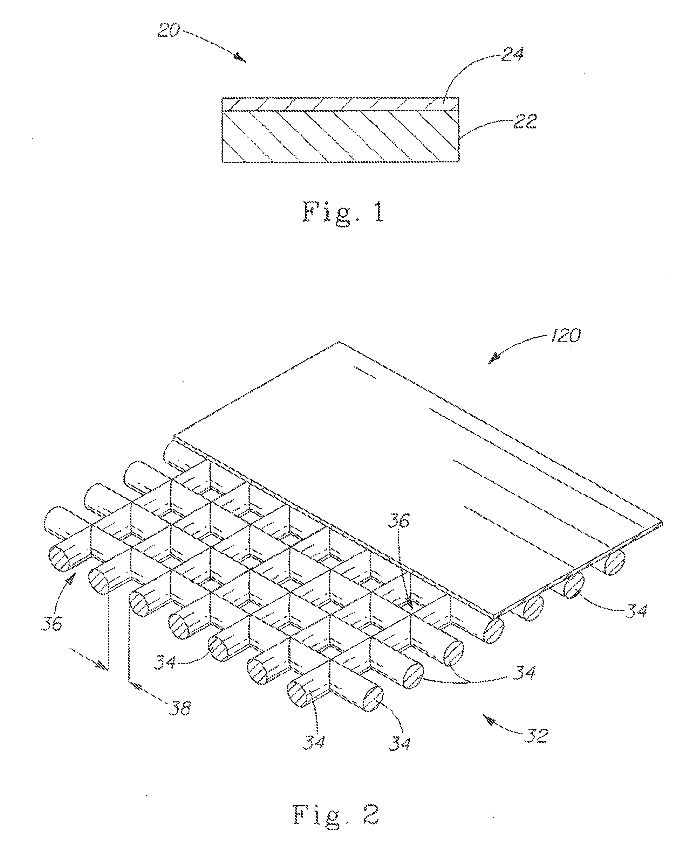

In an embodiment outside of the scope of the present invention, a tooth whitening product 20 comprises a water hydratable polymer film 22 and a solid whitening agent that has been applied as a coating 24 to one side of the water hydratable polymer film 22, as shown in Fig. 1. The solid tooth whitening agent can be applied while the film is still tacky or after the film has dried or cured. Application of the tooth whitening agent in this manner reduces exposure of the tooth whitening agent to any water that may have been used as a solvent in forming the film and also reduces the amount of contact between the water hydratable polymer and the tooth whitening agent in comparison to a water hydratable film having a tooth whitening agent dispersed there throughout. In addition, this embodiment has an increased concentration of the tooth whitening agent at the surface that is applied to the teeth. Such a coating can also assist in the unidirectional diffusion of the tooth whitening agent toward the tooth surface since the coating is directly adjacent and/or in contact with the tooth surface during use. Suitable solid tooth whitening agents that can be applied to one side of a water hydratable polymer film include carbimide peroxide, calcium peroxide, percarbonate, sodium percarbonate, perborates, persulfates, and mixtures thereof. These tooth whitening agents can be sprayed, applied by gravure printing, mist grinding, drop powdering, and other processes known in the art.

The coating of the solid tooth whitening agent can cover all or a portion of the water hydratable polymer film. The thickness of the coating can be greater than about 0.001 microns, or greater than about 0.005 microns, or greater than about 0.01 microns or greater than about 0.05 microns, or greater than about 0.1 microns, or greater than about 1 micron, or greater than 5 microns and/or less than about 100 microns, or less than about 50 microns, or less than about 10 microns, or less than about 1 micron. The dose per unit area of the tooth whitening agent is at least about 0.001 mg/cm², or at least about 0.005 mg/cm², or at least about 0.01 mg/cm², or at least about 0.05 mg/cm², or at least about 0.1 mg/cm², or at least about 1 mg/cm², or at least about 10 mg/cm², or at least about 100 mg/cm² and/or less than about 500 mg/cm², or less than about 250 mg/cm², or less than about 100 mg/cm², or less than about 10 mg/cm². Other materials can be mixed with the solid tooth whitening agent or applied sequentially before or after application of the solid tooth whitening agent. For example, binders, adherents, starches, sweeteners and flavorants, colorants (e.g., to aid in distinguishing the active side of the strip from the non-active side), and other oral care actives can be applied as part of the application step. While the solid tooth whitening agent can be applied in a dry form, it is contemplated that a solvent could be used with the tooth whitening agent during application. In one embodiment, a solvent that solubilizes both the water hydratable polymer and the tooth whitening agent can be employed when applying the tooth whitening agent to the surface of the water hydratable film. In this process, the tooth whitening is solubilized in the solvent and then applied to the surface of the water hydratable film so that the tooth whitening agent is dispersed at least partly within the film as the solvent can solubilize a portion of the film. Alternatively, the solvent may only solublize the tooth whitening agent, in which case a discrete coating would be formed on one side of the film after solvent evaporation or removal. Suitable solvents could include solvents that can solubilize the tooth whitening agent at a given temperature but which do not solubilize the water hydratable polmer at that temperature. Some examples include Cellosolve acetate, anisole, 1,4 dioxane, ethyl acetate, ethylenediamine, dimethyl Cellosolve, Cellosolve solvent, ethanol, Carbitol solvent, n-butanol, cuyl Cellosolve, n-butyl acetate, 2-propanol, and methyl Cellosolve.

The water hydratable polymer film 22 can be prepared using a conventional extrusion, calendaring, pressing or solvent casting processes. For example, to prepare a film by solvent casting polyethylene oxide, the ethylene oxide polymer or mixture of polymers is dissolved in a sufficient amount of a solvent which is compatible with the polymer. Examples of suitable solvents include water, alcohols, acetone, ethyl acetate or mixtures thereof. After a solution has been formed, a plasticizer is added with stirring, and heat is applied if necessary to aid dissolution, until a clear and homogeneous solution has been formed, followed by the addition of the whitening agent and any other ingredients such as flavors. The solution is coated onto a suitable carrier material and dried to form a film. The carrier material must have a surface properties that allow the polymer solution to spread evenly across the intended carrier width without soaking in to form a destructive bond between the two substrates. Examples of suitable carrier materials include glass, stainless steel, teflon, polyethylene-impregnated kraft paper. Drying of the film may be carried out in a high-temperature air-bath using a drying oven, drying tunnel, vacuum drier, or any other suitable drying equipment, after which the tooth whitening agent can be sprayed onto the film.

The efficacy and/or stability of the tooth whitening agent can be increased by forming the water-hydratable film with a solvent other than water so that the tooth whitening agent does not come into contact with water during the formation process and there is no residual water left in the film post manufacture. As used herein, the term "stability" is intended to refer to the propensity of a material to maintain its original concentration or structure over a fixed period of time. As used herein, the term "efficacy" is intended to refer to the amount of tooth whitening per unit time. In one process, the water-hydratable polymer and the tooth whitening agent are mixed and then fed to an extruder whose screw, through mechanical action, melts the water-hydatratable polymer. The melted polymer is then extruded into a film to be formed into the tooth whitening product.

The efficacy of the tooth whitening agent can also be increased by reducing the amount of water hydratable polymer that forms the film In one embodiment, the water hydratable polymer film further comprises water insoluble organic and/or inorganic additives to reduce the amount of the water hydratable polymer so that solubilization of the tooth whitening agent is maximized during use. Suitable water insoluble organic materials include polyolefins (e.g., polyethylene, polypropylene, polybutenes, polyisoprenes, and copolymers thereof) and polyester. Suitable water insoluble inorganic materials include calcium phosphate, calcium pyrophosphate, and titanium dioxide, and silica. The water insoluble additives can comprise at least about 10%, or at least about 20%, or at least about 30% and/or less than about 90%, or less than about 80%, or less than about 70%, or less than about 50% or less than about 40%, or less than about 30% by weight of the film. In these embodiments, the amount of the water hydratable polymer is at least about 5%, or at least about 10%, or at least about 20%, or at least about 30% and/or less than about 90%, or less than about 80%, or less than about 70% by weight of the film. The water insoluble additives can be ground prior to incorporation into the film. In one embodiment, the average particle size of the water insoluble additives is at least about 1 micron, or at least about 20 microns, or at least about 25 microns and/or less than about 100 microns, or less than about 50 microns, or less than about 25 microns, or less than about 10 microns. In addition to decreasing the amount of water hydratable polymer that is available to react with the tooth whitening agent, the concentration of tooth whitening agent available at the surface of the tooth can be increased during hydration, because more water is available to solubilize the tooth whitening agent rather than hydrating or otherwise solubilizing the water hydratable polymer. The tooth whitening agent can be admixed with the water hydratable polymer as described in U.S. patent no. 6,419,906 or coated onto the film as previously described.

In the present invention, a web, scrim, or mesh is incorporated in the tooth whitening product to improve the hydration of the film. The web, scrim, or mesh can be formed from fibers that are aligned in random or repeating geometric patterns. Referring to Fig. 2, a tooth whitening product 120 comprising a web 32 is illustrated. The web 32 is formed from fibers 34 that are arranged in a repeating geometric pattern. The fibers can be formed from one or more water hydratable polymers and may have a tooth whitening agent incorporated therein. Alternatively, the fibers can be formed from water insoluble materials in which case the web will function in a manner similar to the previously described films that incorporate water insoluble materials. The fibers are arranged in a manner to provide void spaces 36 between the fibers. The void spaces can facilitate hydration of the web 32 and therefore solubilization of the tooth whitening agent. The void spaces can vary in size or have a substantially constant size over the web. For example, the void spaces might be smaller in one region and larger in another region depending upon the desired rate of hydration. The fibers 34 can have a diameter of at least 1 micron, or at least 5 microns, or at least 10 microns, or at least 20 microns, or at least 50 microns and/or less than 200 microns, or less than 100 microns, or less than 50 microns, or less than 20 microns. The spacing 38 between fibers is at least 1 micron, or at least 5 microns, or at least 10 microns, or at least 20 microns, or at least 50 microns, or at least 1mm, or at least 1.5mm and/or less than 5 mm, or less than 2.5 mm, or less than 1.5 mm, or less than 50 microns.

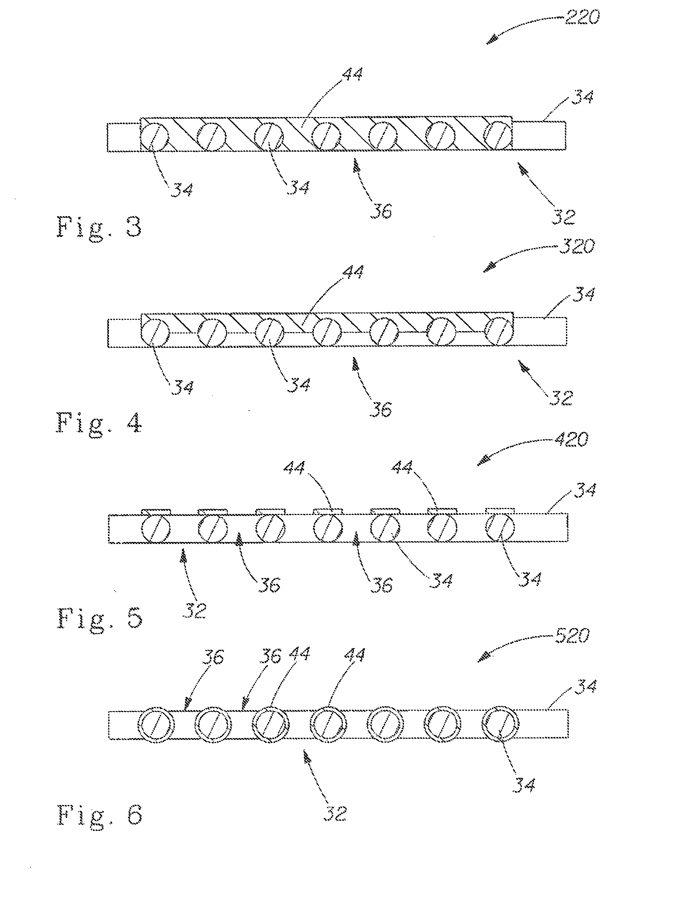

In an alternate embodiment, a tooth whitening product 220, shown in Fig. 3, comprises a web 32 having a coating or layer 44 applied thereto. The web 32 can be formed from a water-hydratable polymer or other material, such as other polymers (e.g., polypropylene, polyethylene, etc.) and cellulose. The fibers 34 of the web 32 can be arranged in a random or repeating pattern. The coating 44 comprises a water-hydratable polymer and a tooth whitening agent. Other materials can be included in the coating, such as a plasticizer, water, water insoluble additives, etc. The coating can bridge the void spaces 36 such that a substantially solid layer is formed on web 32. The coating might completely fill the void spaces as shown in Fig. 3 or partially fill the void spaces as shown in Fig. 4 with respect to tooth whitening product 320. When the void spaces are partially filled, the pocket 50 that is formed can facilitate hydration of the web and therefore solubilization and release of the tooth whitening agent. Alternatively, the coating 44 can only partially bridge the void spaces, as shown in Fig. 5 for the tooth whitening product. 420. In yet another embodiment, a coating 44 does not bridge the void spaces 36 of web 32 but merely coats the fibers 34, either wholly or partially, as shown by way of example in Fig. 6 for the tooth whitening product 520.

The concentration of the tooth whitening agent within the coating and/or the fibers and the amount of water-hydratable polymer can be varied within these web embodiment depending upon the extent to which the coating bridges the void spaces and/or coats the fibers and based upon the desired rate of solubilization of the tooth whitening agent. As will be appreciated, any combination of completely filling the voids, partially filling the voids, partially bridging the voids, and coating the fibers can be provided in one embodiment.

The above described embodiments of the present invention can be further combined with other layers such as a thin protective coating layer, e.g., of 10 nanometers (nm) to 500 microns (um) thickness. The coating material is applied in a sufficiently thin layer so as not to interfere with the flexibility of the film and to allow the whitening strip to conform to an arrangement of a row of teeth. The coating materials can be one or a combination of high molecular weight (that is, molecular weights greater than 1,000,000 Dalton) and include, ethyl cellulose, propyl cellulose, isopropyl cellulose, butyl cellulose, t-butyl cellulose, cellulose acetate, and derivatives of polyvinyl alcohol such as polyvinyl acetate and shellac.

The tooth whitening products of the present invention can be packaged in pouches as individual strips or a roll of film can be provided in a tape-like dispenser, wherein individuals strips can be cut from the roll for use in the oral cavity or the film can be provided with perforations or other frangible features to permit separation of predetermined length strips from the roll of film. Optionally, the tooth whitening products of the present invention can further include a release liner. The release liner can be formed from any material that exhibits less affinity for the film and/or web than the film or web exhibits for itself. The release liner can be formed from polymer films, paper, foils, woven, non-wovens, and other suitable materials known in the art. Optionally, the release liner can include a coating such as wax, silicone, Teflon®, fluoropolymers, etc. The films of the present invention can be formed directly on the release liner. The release liner can be cut to the desired size either before or after formation of the film thereupon. The tooth whitening products of the present invention can also be provided as liner for dental trays, such as those described in U.S. patent no. 5,098,303, wherein the strips are incorporated into the trough of the dental tray.

To use the tooth whitening products of the present invention, the film when applied to the teeth surface when hydrated by saliva in the oral cavity or prewetted by dipping the strip in water will adhere to the teeth in an appropriate manner. In this regard, the tooth whitening product is formed to have a width dimension suitable to cover a row of teeth (upper or lower). Therefore, the tooth whitening product may be applied to the upper set of teeth, or to the lower set of teeth either separately or simultaneously. The length dimension of the tooth whitening product is determined by the amount of coverage desired. In this regard, the number of teeth which it is desired to whiten will determine the dimensions of the product. For instance, it may be desired to only whiten the front teeth, which are most easily seen by others. Accordingly, the length of tooth whitening product can be reduced in this case, as compared to the case where it is desired to whiten all of the teeth. The duration of application of product to the teeth will depend upon the type and concentration of the tooth whitening agent, as well as the type and intensity of extrinsic or intrinsic stain.

The embodiments described herein were chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally and equitably entitled.

## Claims

1. A tooth whitening product, comprising:
a web formed from a plurality of fibers, said fibers arranged to provide a plurality of void spaces; and
a water hydratable polymer coating deposited on said web, said coating including a tooth whitening agent, wherein the coating partially bridges one or more of the plurality of void spaces.

2. A tooth whitening product according to Claim 1, wherein one or more of said plurality of fibers comprise said water hydratable polymer.

3. A tooth whitening product comprising:
a web formed from a plurality of fibers, said fibers arranged to provide a plurality of void spaces; and
a water hydratable polymer coating deposited on said web, said coating including a tooth whitening agent, wherein said coating wholly or partially coats the fibers but does not bridge the void spaces.

4. A tooth whitening product according to Claim 3, wherein one or more of said plurality of fibers comprise said water hydratable polymer.

5. A tooth whitening product according to any of the preceding claims, wherein the water hydratable polymer coating further comprises one or more water insoluble additives and wherein the amount of the water insoluble additives is at least about 30% of weight of the water hydratable polymer coating.

6. A tooth whitening product according to Claim 5, wherein the average particle size of the water insoluble additives is between 1 micron and 100 microns.

## Patentansprüche

1. Zahnaufhellungsprodukt, umfassend:
eine aus einer Vielzahl von Fasern gebildete Bahn, wobei die Fasern so angeordnet sind, dass sie eine Vielzahl von Hohlräumen bereitstellen; und
eine auf der Bahn angelagerte wasserhydratisierbare Polymerbeschichtung, wobei die Beschichtung einen Zahnaufheller aufweist, wobei die Beschichtung einen oder mehrere der Vielzahl der Hohlräume teilweise überbrückt.

2. Zahnaufhellungsprodukt nach Anspruch 1, wobei eine oder mehrere der Vielzahl der Fasern das wasserhydratisierbare Polymer umfassen.

3. Zahnaufhellungsprodukt, umfassend:
eine aus einer Vielzahl von Fasern gebildete Bahn, wobei die Fasern so angeordnet sind, dass sie eine Vielzahl von Hohlräumen bereitstellen; und
eine auf der Bahn angelagerte wasserhydratisierbare Polymerbeschichtung, wobei die Beschichtung einen Zahnaufheller aufweist, wobei die Beschichtung die Fasern ganz oder teilweise beschichtet, jedoch die Hohlräume nicht überbrückt.

4. Zahnaufhellungsprodukt nach Anspruch 3, wobei eine oder mehrere der Vielzahl der Fasern das wasserhydratisierbare Polymer umfassen.

5. Zahnaufhellungsprodukt nach einem der vorstehenden Ansprüche, wobei die wasserhydratisierbare Polymerbeschichtung ferner einen oder mehrere wasserunlösliche Zusatzstoffe umfasst und wobei die Menge der wasserunlöslichen Zusatzstoffe mindestens ungefähr 30 Gew.-% der wasserhydratisierbaren Polymerbeschichtung beträgt.

6. Zahnaufhellungsprodukt nach Anspruch 5, wobei die durchschnittliche Teilchengröße der wasserunlöslichen Zusatzstoffe zwischen 1 Mikrometer und 100 Mikrometern liegt.

## Revendications

1. Produit de blanchissement des dents, comprenant :
une nappe formée d'une pluralité de fibres, lesdites fibres arrangées pour fournir une pluralité d'espaces vides ; et
un revêtement polymère hydratable dans l'eau déposé sur ladite nappe, ledit revêtement incluant un agent de blanchissement des dents, le revêtement reliant partiellement un ou plusieurs parmi la pluralité d'espaces vides.

2. Produit de blanchissement des dents selon la revendication 1, dans lequel une ou plusieurs parmi ladite pluralité de fibres comprendre ledit polymère hydratable dans l'eau.

3. Produit de blanchissement des dents, comprenant :
une nappe formée d'une pluralité de fibres, lesdites fibres arrangées pour fournir une pluralité d'espaces vides ; et
un revêtement polymère hydratable dans l'eau déposé sur ladite nappe, ledit revêtement incluant un agent de blanchissement des dents, ledit revêtement revêtant entièrement ou partiellement les fibres, mais ne reliant pas les espaces vides.

4. Produit de blanchissement des dents selon la revendication 3, dans lequel une ou plusieurs parmi ladite pluralité de fibres comprendre ledit polymère hydratable dans l'eau.

5. Produit de blanchissement des dents selon l'une quelconque des revendications précédentes, dans lequel le revêtement polymère hydratable dans l'eau comprend en outre un ou plusieurs additifs insolubles dans l'eau et dans lequel la quantité des additifs insolubles dans l'eau est au moins environ 30 % du poids du revêtement polymère hydratable dans l'eau.

6. Produit de blanchissement des dents selon la revendication 5, dans lequel la taille moyenne des particules des additifs insolubles dans l'eau est comprise entre 1 micron et 100 microns.
